(19) Europäisches Patentamt
European Patent Office
Office européen des brevets

(11) Publication number: **0 013 836**
**B1**

(12) **EUROPEAN PATENT SPECIFICATION**

(45) Date of publication of patent specification: **28.03.84**

(21) Application number: **79303064.4**

(22) Date of filing: **28.12.79**

(51) Int. Cl.³: **C 08 F 220/28,**
C 08 F 283/06,
C 08 F 246/00, C 09 D 7/00

# (54) Compositions containing acrylic emulsion copolymers and their use as thickeners.

(30) Priority: **29.12.78 US 974466**
**17.09.79 US 75862**
**10.12.79 US 101615**

(43) Date of publication of application:
**06.08.80 Bulletin 80/16**

(45) Publication of the grant of the patent:
**28.03.84 Bulletin 84/13**

(84) Designated Contracting States:
**AT BE CH DE FR GB IT NL SE**

(56) References cited:
**EP - A - 0 003 235**
**EP - A - 0 011 806**
**DE - A - 2 025 696**
**DE - A - 2 438 868**
**US - A - 3 652 497**
**US - A - 4 138 381**

(73) Proprietor: **Rohm and Haas Company**
**Independence Mall West**
**Philadelphia, Pennsylvania 19105 (US)**

(72) Inventor: **Chang, Ching-Jen**
**16 Farber Drive**
**Chalfont, PA 18914 (US)**
Inventor: **Stevens, Travis Edward**
**628 Buckley Road**
**Ambler, PA 19002 (US)**

(74) Representative: **Angell, David Whilton et al,**
**Rohm and Haas Company Patent Department**
**Chesterfield House Bloomsbury Way**
**London WC1A 2TP (GB)**

EP 0 013 836 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Compositions containing acrylic emulsion copolymers and their use as thickeners

This invention relates to compositions containing, as cothickeners, surfactant and emulsion copolymer, to methods of thickening utilizing the compositions, and to other aspects including coating compositions and other aqueous systems thickened with the cothickeners.

Salts of polyacrylic acid and polymethacrylic acid are well known as thickeners for various aqueous systems. A polyacrylic acid obtained by copolymerizing acrylic acid with a small amount (about 0.2 to 1% by weight on the weight of the acrylic acid) of diallyl sucrose (U.S. Pat. 2,798,053) has also been sold for use as a thickener for many years. These thickening agents are difficult to handle because they are either powders that are slow to dissolve or very viscous aqueous solutions. Adverse effects such as stiffness or water sensitivity also may be imparted to the finished product by the polymeric acid thickener. Still another problem associated with the acid thickeners is their electrolyte sensitivity. The aqueous systems thickened with these thickeners decrease drastically in viscosity upon addition of an electrolyte, such as sodium chloride.

British patent 870,994 discloses the preparation of aqueous emulsion copolymers of methacrylic acid and a lower ($C_1$—$C_4$) alkyl acrylate which gives good thickening upon neutralization. The copolymer dispersions having a solids concentration of 25 to 50% by weight, are low viscosity fluids and are thus readily added directly to systems to be thickened. However, they also have severe electrolyte sensitivity.

These polyelectrolyte polymers are useful as bodying and suspending agents in various mucilaginous and colloidal gel-like applications such as dentrifices, surgical jellies, creams and ointments, printing paste thickeners, and the like. However, most polyelectrolyte solutions decrease drastically in viscosity upon the addition of electrolytes such as sodium chloride. These prior art thickener materials are ion-sensitive and do not adequately maintain the viscosities of water or organic solvent solutions containing inorganic salts such as sodium chloride even when a third monomer such as 2-ethylhexylacrylate or styrene respectively is included in the polymer as is suggested by the respective prior art patents.

U.S. Patent 4,138,381 discloses at column 2, lines 7 to 41, and column 12, claim 1, lines 2 to 43, a composition as follows:

"A liquid composition useful as a thickening agent in polymeric latices, the composition comprising
(A) up to 50% by weight of the composition, of a polymer of polymerized units of
  (1) about 10—98%, by weight of the polymers, at least one unsaturated carboxylic acid of 3—6 carbon atoms;
  (2) about 1—50%, by weight of the polymer, at least one alkyl acrylate or alkyl methacrylate whose alkyl group contains 1—30 carbon atoms; and
  (3) about 1—85%, by weight of the polymer, at least one ester of the formula

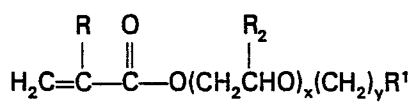

  where
    R and $R_2$ are hydrogen or methyl;
    $R_1$ is an alkyl radical or an alkyl phenyl radical of 1—20 carbon atoms;
    x is 5—80; and
    y is 0—20; the total of 1, 2 and 3 being 100%; and
(B) as a solvent for (A),
  (4) a glycol, or
  (5) a glycol containing up to 50% of its weight of water; the composition being made by
    (a) adding 10—50%, by weight of the total weight of monomers to be used, to a reaction vessel containing enough glycol, or glycol containing up to 50% of its weight of water, to give a final polymer concentration of less than 50% by weight;
    (b) heating the contents of the vessel to at least 50°C.;
    (c) adding a portion of the free-radical catalyst to be used to the vessel; and then
    (d) continuously adding the remainder of the monomers and catalyst to the vessel, and
    (e) adding ammonia or a water-soluble organic amine after the polymerization is complete to adjust the pH to about 7 to 10."
(Column 12, claim 1, lines 2 to 43.)

In accordance with the present invention, stable aqueous dispersions of certain water-insoluble, emulsion copolymers of methacrylic acid (MAA) are produced which are quite fluid at a pH below about 7 even though they have solids contents of 25 to 50% or even higher, but upon partial or substantial neutralization with ammonium hydroxide or an alkali metal hydroxide, such as sodium,

potassium, or lithium hydroxide, or a volatile amine, such as triethylamine or triethanolamine, become highly viscous and are suitable for thickening aqueous media of a wide variety, using the same general procedure disclosed in the British patent mentioned above. As compared to the thickeners of the British patent, those copolymer thickeners of the present invention containing the polymerized units of (meth)acrylic acid ester of a ($C_8$—$C_{30}$)alkyl, alkylaryl, or polycyclic alkyl mono ether of a polyethylene glyol defined herein generally provide markedly greater viscosity at given levels, and have one or more advantages, such as less sensitivity to electrolyte content of the aqueous medium thickened; also improved flow and leveling when used as a rheology modifier in latex coating compositions such as water-base paints, in pigment printing pastes for coloring of textiles the use of thickeners of the present invention is quite advantageous in respect of ease of formulation, compatibility with a wide variety of pigment binders and pigment dispersions, high thickening efficiency resulting in brilliant coloration, good color yield and sharp demarkation, freedom from flushing and haloing, minimal stiffening of the printed area, resistance to crocking, to washing, to dry cleaning, and to exposure to sunlight. In "all-aqueous" silk screen pigment printing systems, the thickeners of the present invention provide convenience of handling, good color depth, sharp mark detail with no haloing, and improved "holdout", i.e. less "strikethrough".

EP—A—79 104600.6 discloses copolymers of vinyl surfactant esters, carboxylic acids and ethylenically unsaturated monomers some of which overlap with the polymers (A) in the present invention. However these prior art copolymers are not disclosed in compositions cothickened with the specified amounts of surfactant of this invention or at all for use in a pigment printing composition, dentifrice, hand lotion, pharmaceutical, household detergent or fracturing fluid.

US—A—3652497 discloses polymeric surfactant thickeners based on copolymers of 5 to 30% carboxylic monomers, optionally unsaturated non-acid monomers, and as distinct from the polymer of present invention 70—95% non-ionic vinyl surfactant ester. DE—A—2025696 discloses thickener compositions based on polymers of vinyl surfactant esters, methacrylic acid and as distinct from the present invention, styrene and/or butadiene. The thickeners of these two disclosures have not proved to be sufficiently effective to be commercially attractive.

The compositions of the invention comprise thickener and/or thickened compositions containing certain thickener copolymer and surfactant in specified proportions.

The thickener copolymers used in the compositions of the present invention are those obtainable by aqueous emulsion copolymerization of monomers falling within the following three or four classes:

(1) methacrylic acid or acrylic acid sometimes hereinafter referred to as MAA, or AA, respectively

(2) an acrylic or methacrylic acid ester of a ($C_8$—$C_{30}$) alkyl, alkylaryl, or polycyclic hydrocarbyl monoether of a polyethylene glycol having at least 2 oxyethylene units therein, preferably having at least 10 to 40 (or more up to 70 or so) oxyethylene units. This monomer may be defined by the general formula:

$$H_2C{=}C(R){—}C(O){—}O{—}(CH_2CH_2O)_n{—}R^o \qquad (I)$$

wherein

R is H or $CH_3$, the latter being preferred,

n is at least 2, and preferably has an average value of at least 10, up to 40 to 60 or even up to 70 or so, (in the case of a single compound n is an integer having the specified value, in the case of a mixture of compounds n is an average number), and

$R^o$ is a hydrophobic group, for example, an alkyl, alkylaryl, and polycyclic alkyl group having 8 to 30 carbon atoms, preferably 12 to 24, such as 12 to 20 or 12 to 18, more preferably 16 to 18. $R^o$ represents a single radical or a mixture of radicals having an average number of carbon atoms in the specified ranges.

(3) A ($C_1$—$C_4$)alkyl acrylate or alkyl methacrylate, preferably ethyl acrylate, butyl acrylate or methyl methacrylate, sometimes referred to hereinafter as (EA), (BA), or (MMA), respectively, most preferably EA, and

(4) Optionally, a small amount of a polyethylenically unsaturated monomer.

In general, the emulsion copolymer dispersions obtained have solids contents of from 25 to 50% by weight and the three-component copolymer dispersion has a weight average molecular weight of about 100,000 to several million. A chain-transfer agent may be used to obtain molecular weights in the lower part of the range or even down to about 80,000. On the other hand, use of 0.05% to about 1.0% of monomer component (4), based on total monomers, serves to provide molecular weights in or above the higher portion of the range mentioned.

The relative proportions of the first three components fall in the broad range of (1) 20—69.5 weight percent, (2) 0.5 to 25 weight percent, and (3) at least 30 weight percent, the total percentages of the three components being 100. The preferred ranges are (1) 20—50%, more preferably 30—50%, such as 35—45%, most preferably 30—45%, (2) 2—25% or 1—15%, such as 5—15%, (3) at

least 40%, more preferably 40—60%. In component (2) $R^o$ may be octyl ($C_8$), lauryl ($C_{12}$), tridecyl ($C_{13}$), myristyl ($C_{14}$), or pentadecyl ($C_{15}$), but it is preferably $C_{12}$ to $C_{18}$ or mixtures thereof, examples being lauryl, cetyl, palmityl, and stearyl. $R^o$ may be longer chain alkyl groups such as eicosyl ($C_{20}$). $R^o$ may be alkylaryl, such as octyl- and vinylphenyl, or polycyclic alkyl such as cholesterol and lanosterol. A mixture of several ($C_8$—$C_{30}$) alkyl ethers may be used.

The emulsion thickener copolymers may be produced by conventional aqueous emulsion polymerization techniques, using appropriate emulsifiers for emulsifying the monomer and for maintaining the polymer obtained in a stable, dispersed condition. Commonly used anionic surfactants such as sodium lauryl sulfate, dodecylbenzene sulfonate and ethoxylated fatty alcohol sulfate can be used as emulsifiers. The emulsifier may be used in a proportion of 1/2 to 6% of the weight of monomers.

Preferably, water-soluble initiators such as alkali metal or ammonium persulfate are used in amounts from 0.01 to 1.0% on the weight of monomers. A gradual addition thermal process employed at temperatures between 60°C to 100°C is preferred over redox system for better incorporation of the hydrophone-containing monomer (component (2)).

The polymerization system may contain small amounts (0.01 to 1% by weight, based on monomer weight) of the chain transfer agent mercaptans such as alkyl mercaptans containing from about 4 to 22 carbon atoms. The use of mercaptan modifier reduces the molecular weight of the polymer and therefore its thickening efficiency. This may be desirable in certain areas of applications where proper rheology but not the thickening efficiency is of primary concern.

The copolymers hereinabove defined may further be modified by introducing a small amount of component (4), namely, a polyethylenically unsaturated copolymerizable monomer effective for crosslinking, such as diallylphthalate, divinylbenzene, allyl methacrylate, or ethyleneglycol dimethacrylate. Thus, from 0.05 to 1.0% of such polyethylenically unsaturated compound based on total weight of monomer may be included in the composition forming the polymer. The resulting copolymers are either highly branched or in the form of three-dimensional networks. In the neutralized salt form, those networks swell in an aqueous system and the consequent "micro-gel" structure acts as a highly efficient thickener.

In the compositions of the invention the emulsion thickener copolymer is used in a mixture with 0.05 to 1.0 parts by weight of surfactant per part of copolymer thickener solids to give a remarkable enhancement of thickening (hereinafter termed "cothickening").

The copolymer may be utilized in a variety of ways to provide the thickener or thickened compositions of the invention for example, the copolymer, while in aqueous dispersion or slurry or dry form, may be blended into an aqueous system to be thickened followed by addition of a neutralizing agent. Alternatively, the copolymer may first be neutralized in aqueous dispersion form and then blended with the aqueous system. The other components may also be separately blended (as dry components or as dispersions or slurries) into the aqueous dispersion to be thickened, before the neutralization step. Although this invention extends to aqueous concentrates of the copolymer in acid form and the surfactant which may be added to an aqueous dispersion to be thickened as needed, followed by neutralization, such concentrates tend to be too viscous for easy handling. It is nevertheless possible to prepare either a dry blend or an aqueous, high solids composition which is sufficiently low in viscosity as to be pumpable or pourable, and then to further thicken the admixture by addition of an alkaline material.

The copolymer thickener may be provided in a dry state in a number of ways. For example, the unneutralized copolymer may be spray or drum dried for subsequent blending with the surfactant cothickener. However, it is also possible to spray dry or otherwise dehydrate the neutralized copolymer thickener, and then reconstitute the aqueous thickener dispersion at a future time and place by agitation in an aqueous medium, provided the pH of the dispersion is maintained at pH 7 or higher.

The more usual method of application of the dispersion of the present invention for aqueous thickening is to add the surfactant and the aqueous dispersion of the carboxylic acid copolymer to the medium to be thickened and, after mixing, to introduce an alkaline material to neutralize the acid. The major portion of the thickening effect is obtained in a few minutes upon neutralization. In the presence of high concentrations of electrolytes, the viscosity development may take much longer. This method of applying a copolymer emulsion to an aqueous system before neutralization enables one to handle a high solids thickener in a non-viscous state, to obtain a uniform blend, and then to convert to a highly viscous condition by the simple addition of an alkaline material to bring the pH of the system to 7 or above.

The aqueous solutions thickened with the cothickeners of this invention exhibit good viscosity stability even at pH as high as 13.

The thickeners described here are useful in a variety of aqueous systems such as textile printing pastes, latex paint formulations, and cosmetic formulations.

The cothickening referred to above has been observed upon the addition of surfactant to an aqueous system containing the emulsion copolymer of the invention, when the emulsion copolymer is neutralized. In some cases the thickening can be enhanced up to about 40 times the viscosity afforded by the neutralized copolymer alone. A wide range of surfactant type and amount is effective. The

surfactant is used in an amount of 0.05 to 1.0 parts by weight of surfactant per part copolymer solids, preferably 0.1 to 0.5 parts surfactant per part copolymer, same basis. Although trace amounts of surfactant may be residually present from the emulsion polymerization of the monomers comprising the emulsion copolymer (for example, whatever may remain of the about 1.5 wt.% surfactant on monomers), such amounts of surfactant are not believed to result in any measurable cothickening.

On the basis of an aqueous system containing about 0.1 to 5% by weight of copolymer solids, a useful amount of surfactant for optimum cothickening is about 0.1 to 1.0% by weight of the total system. As indicated, the optimum amounts of copolymer and surfactant cothickener depend on copolymer and surfactant type and other components of the aqueous system to be thickened. However, it has been observed that cothickening reaches a maximum as surfactant is added and then decreases. Hence, the optimum concentration and copolymer/surfactant ratio can be determined in a routine manner in each case.

The preferred method of application of the emulsion copolymer and the surfactant for aqueous thickening is to add in any sequence the copolymer and the surfactant to the medium to be thickened and, after mixing, to introduce an alkaline material to neutralize the acid. This method of applying copolymer emulsion and surfactant to an aqueous system before neutralization enables one to handle a high solids thickener in a non-viscous state, to obtain a uniform blend, and then to convert to a highly viscous condition by the simple addition of an alkaline material to bring the pH of the system to 7 or above. However, the copolymer in the aqueous system may also be neutralized before addition of the surfactant.

The surfactants which may be used include nonionics and anionics, singly or in combination, the selection necessarily depending upon compatibility with other ingredients of the thickened or thickenable dispersions of the invention. Cationic and amphoteric surfactants may also be used provided they are compatible with the copolymer and other ingredients of the aqueous system, or are used in such small amounts as not to cause incompatibility.

Suitable anionic surfactants that may be used include the higher fatty alcohol sulfates such as the sodium or potassium salt of the sulfates of alcohols having from 8 to 18 carbon atoms, alkali metal salts or amine salts of higher fatty acid having 8 to 18 carbon atoms, and sulfonated alkyl aryl compounds such as sodium dodecyl benzene sulfonate. Examples of nonionic surfactants include alkylphenoxypolyethoxyethanols having alkyl groups of about 7 to 18 carbon atoms and about 9 to 40 or more oxyethylene units such as octylphenoxypolyethoxyethanols, dodecylphenoxypolyethoxyethanols; ethylene oxide derivatives of long-chain carboxylic acids, such as lauric, myristic, palmitic oleic; ethylene oxide condensates of long-chain alcohols such as lauryl or cetyl alcohol, and the like.

Examples of cationic surfactants include lauryl pyridinium chloride, octylbenzyltrimethylammonium chloride, dodecyltrimethylammonium chloride, condensates of primary fatty amines and ethylene oxide, and the like. Amphoteric surfactants include cocoa-beta-alanine, methyl ester of dimethyl octadecyl betaine chloride, and quaternary amine derivatives of fatty acids, such as cetyldimethylammonium inner carboxylate, and the like.

The foregoing and numerous other useful nonionic, anionic, cationic and amphoteric surfactants are described in the literature, such as "McCutcheon's Detergents & Emulsifiers 1978 Annual, North America Edition", MC Publishing Company, Glen Rock, NJ 07452 U.S.A., incorporated herein by reference.

A remarkable enhancement of flow and leveling properties associated with the use of the co-thickeners whereby high film build aqueous coatings are obtained has been observed. This characteristic permits the use of the co-thickeners as rheology modifiers in latex coating compositions such as water base paints. A difficult problem heretofore encountered in designing aqueous coatings having desirable flow and film build properties has been the want of an efficient thickener having low pseudoplasticity which is manifest in a workable low shear viscosity in the range of about 70 to 100 Krebs Units (KU) and, concommitantly, a sufficient high shear viscosity (measured by an ICI cone and plate viscometer) in the range of 0.12 to 0.25 or more Pa · s thereby providing brush drag and film build. Thickeners known in the prior art, while suitable for some applications of coating compositions in which they are contained, offer advantages in certain of the following properties while sacrificing others of the same group of properties: thickening efficiency (that is, minimization of attrition of film resistance), low pseudoplasticity (only slight shear thinning), reasonable expense, phase separation, high ICI viscosity values when used at low levels, sensitivity to electrolytes and charged-particles in solution. The use of low levels of the co-thickeners of the invention overcomes the deficiencies of the prior art compositions and provides thickener compositions which offers a high advantageous balance of thickening efficiency and low pseudoplasticity.

For use as rheology modifier for aqueous coating compositions, low levels, from about 0.5 to 5% by weight, of hydrophobe-containing monomer,

$$H_2C{=}C(R){-}C(O){-}O{-}(CH_2CH_2O)_n{-}R^o,$$

represented hereinabove as of general formula I, contains 8 to 14 carbon atoms, are employed in producing the thickener copolymers used in the compositions of the invention. Preferably, from about 1

to 5% by weight of the hydrophobe-containing comonomer is used. Most preferably, about 3% by weight of the hydrophobe-containing monomer wherein R° is a $C_{12}$ alkyl group is employed.

The thickeners of the invention are also useful in latex paint, dentrifrice, hand lotion, household liquid detergent, "fracturing fluid" pigment dispersion, oil well drilling fluid, textile printing compositions such as pastes or pharmaceuticals.

In another embodiment, the invention provides a method of thickening an aqueous system which comprises providing in the system at least partially neutralized thickener copolymer as described and at least one surfactant in the stated amount.

This invention also extends to the use of the copolymer thickeners *per se*, either without surfactant, or more usually with surfactant outside the range herein defined for cothickening in the following compositions: dentrifrice, pigment printing compositions, household liquid detergent formulations, hand lotions, pharmaceuticals, fracturing fluids.

In the following examples illustrative of the invention, the parts and percentages are by weight and the temperatures are in Celsius degrees unless otherwise stated.

The following Example A is a suitable procedure for producing the acrylic acid and the methacrylic acid ester constituting component (2) but other ways may be employed such as transesterification of a monomeric alkyl acrylate or methacrylate, illustrated by the following Example B, such as ethyl acrylate or methyl methacrylate, with the ($C_8$—$C_{24}$) alkyloxy polyethoxy ethanol.

Example A

A reactor equipped with a condenser and a Dean-Stark trap is charged with 2140 g of an octadecyloxypoly(ethyleneoxy)ethanol containing an average of 20 oxyethylene units, 207 g of methacrylic acid, 1000 g of toluene, 17.4 g of concentrated sulfuric acid, and 4.6 g of monomethylether of hydroquinone. The mixture is stirred and heated to reflux. The water, formed during the esterification, is removed as water/toluene azeotrope with toluene returned to the reactor. The reaction is carried out in contact with air to inhibit polymerization. When no more water is distilled, the mixture is cooled, neutralized with sodium carbonate, filtered, and solvent is stripped to give octadecyloxypoly(ethyleneoxy) ethyl methacrylate as a tan waxy solid. This product is Monomer No. 8 in the following Table I-A.

In the following Table I-A, nineteen monomeric methacrylic acid esters and nineteen acrylic acid esters used to make the emulsion copolymer thickeners, are listed, the third and fourth columns indicating the alkyl group, R° and the value of n in the formula (I) for the higher alkyl ether of the polyethylene glycol (or mixture thereof) that is esterified with the MAA or AA in the procedure of Example A in the first column of Table I-A. The monomers numbered 1 to 19 are methacrylates whereas those in the second monomer number column having numbers 21 through 39 are acrylates obtained by using acrylic acid in place of methacrylic acid in Example A.

TABLE I-A

| Monomer No. | | Alkyl monoether of polyethylene glycol | |
|---|---|---|---|
| Methacrylate | Acrylate | Alkyl | n (No. of EO units) |
| 1 | 21 | Lauryl ($C_{12}$)* | 4 |
| 2 | 22 | Lauryl ($C_{12}$)* | 23 |
| 3 | 23 | ($C_{14}$—$C_{18}$)** | 20 |
| 4 | 24 | ($C_{14}$—$C_{18}$)** | 30 |
| 5 | 25 | ($C_{14}$—$C_{18}$)** | 40 |
| 6 | 26 | Stearyl ($C_{18}$) | 2 |
| 7 | 27 | Stearyl ($C_{18}$) | 10 |
| 8 | 28 | Stearyl ($C_{18}$) | 20 |
| 9 | 29 | ($C_{20}$—$C_{24}$)*** | 20 |
| 10 | 30 | ($C_{20}$—$C_{24}$)*** | 60 |
| 11 | 31 | Stearyl ($C_{18}$) | 30 |
| 12 | 32 | Octylphenyl ($C_{14}$) | 16 |
| 13 | 33 | Octylphenyl ($C_{14}$) | 30 |
| 14 | 34 | Octylphenyl ($C_{14}$) | 40 |
| 15 | 35 | *n*-tridecyl ($C_{13}$) | 13 |
| 16 | 36 | Iso-hexadecyl ($C_{16}$) | 20 |
| 17 | 37 | Lanolin ($C_{30}$) | 25 |
| 18 | 38 | Cholesterol ($C_{27}$) | 24 |
| 19 | 39 | Nonylphenol ($C_{15}$) | 15 |

*Mixture of about 65% n-dodecyl and about 35% n-tetradecyl

**Mixture of monoalkyl ethers (0—4% $C_{14}$, at least 60% $C_{18}$ and at least 23% $C_{16}$)

***Mixture of monoalkyl ethers (60% $C_{20}$, 20% $C_{22}$, 10% $C_{24}$, remaining 10% of higher chain length alkyl, e.g. $C_{25}$—$C_{30}$ and lower chain length alkyl, e.g. $C_{16}$—$C_{18}$).

Example B

To a two liter, 4-necked flask fitted with a stirrer, thermometer, dry air sparge, and distillation head atop an Oldershaw column is charged 786 g (0.5 moles) of n-dodecyloxy poly(ethyleneoxy)ethanol containing an average of 30 oxyethylene units, 400 g (4.0 moles) of methyl methacrylate, and 1.7 g of monomethyl ether of hydroquinone. The mixture is stirred and heated to reflux and water azeotropically distilled. The mixture is then cooled to 60°C and 6.21 g of dibutyltinoxide (catalyst) is charged. The mixture is again heated to reflux. As the transesterification proceeds and the methanol is generated, the head temperature decreases from 100°C to 64°C (MMA/methanol azeotrope). The distillate is collected over 4 hr period until the head temperature rose to 100°C. The mixture is then vacuum stripped to remove the excess methyl methacrylate to give n-dodecyloxypropyl $(ethyleneoxy)_{30}$ methacrylate as a waxy solid. The product is Monomer #46 in Table I-B.

Following the procedure of Example B, nine other n-alkyloxypoly$(ethyleneoxy)_n$ethyl methacrylates and two alkylphenoxypoly$(ethyleneoxy)_n$ethyl methacrylates are prepared and used to make emulsion copolymer thickeners. These monomers are listed in Table I-B.

TABLE I-B
Alkyloxy & Alkylphenoxypoly$(ethyleneoxy)_n$ethanol

| Monomer No. | Alkyl or alkydphenyl | n (No. of EO units) |
|---|---|---|
| 41 | n-Octyl | 30 |
| 42 | n-Decyl | 30 |
| 43 | n-Decyl | 50 |
| 44 | n-Dodecyl | 10 |
| 45 | n-Dodecyl | 20 |
| 46* | n-Dodecyl | 30 |
| 47** | n-Dodecyl/n-Tetradecyl | 23 |
| 48 | n-Tetradecyl | 30 |
| 49 | Octylphenyl | 30 |
| 50 | Nonylphenyl | 15 |
| 51 | n-Octadecyl | 20 |
| 52 | n-Tetradecyl | 0 |

*Example B
**Mixture of about 65% n-dodecyl and about 35% n-tetradecyl.

Example 1

a) An emulsion of monomers in water is prepared by adding 175 g of ethyl acrylate, 140 g of methacrylic acid, 35 g of octadecyloxypoly$(ethyleneoxy)_{20}$ethyl methacrylate from example A (monomer #8 in Table I), 46.7 g of 30% solution of anionic surfactant (the ammonium salt of nonylphenoxypoly(9)ethyleneoxyethanol sulfate, such as that commercially available under the name Alipal EP-110), and 433 g of water. To a reaction vessel containing 308 g of water at 90°C is added 18.8% of the monomer emulsion and 5.5 g of 0.25% ammonium persulfate. After the initial charge had polymerized as evidenced by no refluxing at 90°C, the remaining monomer emulsion and 49.5 g of 0.25% ammonium persulfate are gradually added over a period of one hour. The temperature of the mixture is maintained at 86—90°C. After completion of monomer and initiator feed, the mixture is held at 90°C for 15 minutes and then 16 g of 0.87% ammonium persulfate solution is added and the mixture is then cooled and filtered. The filtrate gives an approximately 30% solids emulsion copolymer dispersion (essentially 100% yield) in which the copolymer composition is 10% octadecyloxypoly$(ethyleneoxy)_{20}$ethyl methacrylate, 50% ethyl acrylate, and 40% methacrylic acid. This dispersion is Polymer C in the following Table II and is thickener composition of the present invention.

b) A series of additional latices are prepared by following the procedure and using a recipe corresponding to that specifically described in part a) except that either a different component (2) monomer of Table I is used in place of that (monomer #8 in Table I) used in part a) or a different weight ratio is used between the several monomers copolymerized to form the polymers of the present invention designated by the letters A through R in the first column of Table II, the second column indicating the monomers used to make the respective polymers, the first of the three being a monomer listed in Table I and designated by the monomer number assigned to it in Table I. The second monomer given is a component (3) monomer, EA representing ethyl acrylate, MA, methyl acrylate; and BA, butyl acrylate. The third monomer given is MAA. The third column of Table II gives the weight ratios of the several monomers in the copolymer. The fourth column in Table II gives the viscosity of the aqueous dispersion of the copolymer upon neutralization with one equivalent of sodium hydroxide, at 1% polymer solids. The viscosity listed is Brookfield at 12 rpm at 23.9°C (75°F) using spindle number 4.

For comparative purposes, a copolymer of 60% EA and 40% MAA made by the procedure of part a) is included in the Table II as Polymer S. On neutralization with one equivalent of NaOH, a Brookfield

viscosity at 1% polymer solids and 23.9°C (75°F), using spindle number 2 at 12 rpm, is 125 mPa · s (centipoises). This is reasonably consistent with the 315 mPa · s (cps.) viscosity of the 60% EA/40% MAA copolymers of British patent 870,994, Table I, page 8, line 11.

TABLE II

| Polymer | Monomers | Weight ratio | 1% Viscosity, mPa · s |
|---|---|---|---|
| A | #2/EA/MAA | 10/50/40 | 2,300 |
| B | #1/EA/MAA | 10/50/40 | 4,250 |
| C | #8/EA/MAA | 10/50/40 | 31,500 |
| D | #8/EA/MAA | 7.5/52.5/40 | 12,400 |
| E | #28/EA/MAA | 5/55/40 | 10,000 |
| F | #27/EA/MAA | 2/58/40 | 6,300 |
| G | #29/EA/MAA | 10/55/35 | 21,000 |
| H | #8/EA/MAA | 10/60/30 | 16,500 |
| I | #7/EA/MAA | 10/50/40 | 15,000 |
| J | #7/EA/MAA | 7.5/52.5/40 | 23,500 |
| K | #7/MA/MAA | 5/55/40 | 8,000 |
| L | #7/EA/MAA | 2/58/40 | 7,000 |
| M | #7/EA/MAA | 7.5/42.5/50 | 21,000 |
| N | #7/EA/MAA | 7.5/47.5/45 | 15,000 |
| O | #7/MA/MAA | 7.5/57.5/35 | 10,000 |
| P | #7/EA/MAA | 7.5/62.5/30 | 6,700 |
| Q | #6/EA/MAA | 10/50/40 | 6,500 |
| R | #8/BA/MAA | 10/50/40 | 19,500 |
| S | EA/MAA | 60/40 | 125 |

c) A series of latices CT through CY are prepared by repeating Example 1 a) specifically except that a component (4) monomer is included in the monomer emulsion. Table III lists these polymers, the component (4) monomer and the amount thereof as well as the viscosity obtained by neutralization with one equivalent of NaOH.

TABLE III

| Polymer | Component (4) monomer | Wt.% | % Viscosity[1] mPa · s |
|---|---|---|---|
| CT | allylmethacrylate | 0.2 | 4,600 |
| CU | trimethylolpropane triacrylate | 0.2 | 15,300 |
| CV | diallyl phthalate | 0.2 | 16,000 |
| CW | ethylene glycol dimethacrylate | 0.2 | 22,000 |
| CX | ethylene glycol dimethylacrylate | 0.05 | 15,000 |
| CY | ethylene glycol dimethacrylate | 0.02 | 17,500 |

[1]Brookfield viscosity at 12 rpm at 23.9°C (75°F) spindle No. 4

Example 2

a) An emulsion of monomers in water is prepared by mixing 118 g of ethyl acrylate, 94.5 g of methacrylic acid, 23.7 of cetyl-stearyloxypoly(ethyleneoxy)$_{20}$ethyl methacrylate (monomer #3 of Table I) 15.7 g of 30% solution of Alipal EP-110, and 270 g of water. To a reaction vessel containing 7.9 g of 30% solution of Alipal EP-110 in 208 g of water at 90°C there is added 10% of the monomer emulsion and 7.0 g of 0.25% ammonium persulfate solution. After the initial charge had polymerized as evidenced by no refluxing at 90°C the remaining monomer emulsion and 30.0 g of 0.25% ammonium persulfate solution are gradually added over a period of one hour at 86—90°C. After completion of monomer and initiator feed, the mixture is held at 90°C for 15 minutes and then 23.4 g of 0.38% ammonium persulfate solution is gradually added over 20 minutes. After another 30-minute hold at 90°C, the mixture is cooled and filtered. The filtrate gives an approximately 30% solids emulsion copolymer dispersion in which the copolymer composition is 10% cetylstearyloxypoly(ethyleneoxy)$_{20}$ethyl methacrylate, 50% ethyl acrylate and 40% methacrylic acid (Polymer AB of Table IV).

b) A series of additional latices are prepared by following the procedure and using a recipe similar to the one described in part a) of this Example 2 except that either a different component (2) monomer (having the monomer number in Table I) and/or the monomer ratios are changed. the aqueous viscosities observed upon neutralization with one equivalent of sodium hydroxide at 1% polymer solids are shown in Table IV. (Brookfield viscosities at 23.9°C (75°F), using spindle No. 4).

TABLE IV

| Polymer | Monomers | Wt. ratio | 1% Viscosity, mPa · s 12 rpm | 1.5 rpm |
|---|---|---|---|---|
| Z | #3/EA/MAA | 5/55/40 | 13,000 | — |
| AA | #3/EA/MAA | 7.5/52.5/40 | 25,000 | 90,000 |
| AB | #3/EA/MAA | 10/50/40 | >50,000 | 354,000 |
| AC | #3/EA/MAA | 15/45/40 | 31,500 | 176,000 |
| AD | #4/EA/MAA | 5/55/40 | 8,000 | — |
| AE | #4/EA/MAA | 7.5/52.5/40 | 32,000 | — |
| AF | #4/EA/MAA | 10/50/40 | >50,000 | 222,000 |
| AG | #4/EA/MAA | 15/45/40 | >50,000 | 400,000[1] |
| AH | #5/EA/MAA | 5/55/40 | 19,750 | — |
| AI | #5/EA/MAA | 7.5/52.5/40 | 38,000 | 59,000 |
| AJ | #5/EA/MAA | 10/50/40 | >50,000 | 208,000 |
| AK | #5/EA/MAA | 15/45/40 | >50,000 | 336,000 |
| AL | #8/EA/MAA | 10/50/40 | 30,000 | — |
| AM | #9/EA/MAA | 5/55/40 | >50,000 | 216,000 |
| AN | #9/EA/MAA | 7.5/52.5/40 | >50,000 | 400,000[2] |
| AO | #9/EA/MAA | 10/50/40 | >50,000 | 240,000 |
| AP | #3/EA/MAA | 10/60/30 | 25,000 | — |
| AQ | #3/EA/MAA | 10/65/25 | 22,000 | — |
| AR | #3/EA/MAA | 10/70/20 | 10,000 | — |
| AS | #3/EA/MAA | 10/75/15 | 700 | — |
| AT | #3/EA/MAA | 10/80/10 | 75 | — |
| AU | #3/EA/AA | 10/70/20 | 1,250 | — |
| AV | #3/EA/AA | 10/75/15 | 300 | — |
| AW | #3/EA/AA | 10/80/10 | 40 | — |

[1]820,000 mPa · s at 0.6 rpm
[2]980,000 mPa · s at 0.6 rpm

Example 3

Following the procedure and recipe of Example 2 a), a series of latices are prepared in the presence of chain transfer agent mercaptans. Their aqueous viscosity data upon neutralization with one equivalent of sodium hydroxide at 3% polymer solids are shown in Table V.

TABLE V

| Polymer | Monomers | Wt. ratio | Mercaptan[1] | Wt.% | 3% Visc.[2] mPa · s | Spindle No. |
|---|---|---|---|---|---|---|
| AX | #3/EA/MAA | 2/58/40 | DDM | 0.33 | 190 | 3 |
| AY | #3/EA/MAA | 2/58/40 | DDM | 1.32 | 40 | 2 |
| AZ | #3/EA/MAA | 2/58/40 | MPA | 0.10 | 460 | 3 |
| BB | #3/EA/MAA | 2/58/40 | MPA | 0.68 | 40 | 2 |
| BC | #3/EA/MAA | 5/55/40 | DDM | 0.33 | 2,770 | 4 |
| BD | #3/EA/MAA | 5/55/40 | DDM | 1.32 | 40 | 2 |
| BE | #3/EA/MAA | 5/55/40 | MPA | 0.10 | 9,100 | 4 |
| BF | #3/EA/MAA | 5/55/40 | MPA | 0.68 | 140 | 2 |

[1]DDM=dodecyl mercaptan
MPA=mercaptopropionic acid
[2]Brookfield Viscosity at 60 rpm at 23.9° (75°F) using spindle number indicated.

Example 4

a) An emulsion of monomers in water is prepared by mixing 118 g of ethyl acrylate, 94.5 g of methacrylic acid, 23.7 g of cetyl-stearyloxypoly(ethyleneoxy)$_{30}$ethyl methacrylate (monomer 4 of Table I), 6.3 g of 28% sodium lauryl sulfate, and 270 g of water. To a reaction vessel containing 6.3 g of 28% sodium lauryl sulfate in 208 g of water at 86°C there is added 5% of the monomer emulsion and 20.3 g of 1.25% ammonium persulfate solution. After the initial charge has polymerized at 86°C, the remaining monomer emulsion and 8.7 g of 1.25% ammonium persulfate solution are gradually added over a period of one hour at 86°C. After completion of monomer and initiator feed, the mixture is held at 86°C for 15 minutes and then 30.0 g of 0.3% ammonium persulfate solution is added. After another 75-minute hold at 86°C, the mixture is cooled and filtered. The filtrate is an approximately 30% solids

emulsion copolymer dispersion in which the copolymer composition is 10% cetyl-stearyl-(ethyleneoxy)$_{30}$ethyl methacrylate, 50% ethyl acrylate, and 40% methacrylic acid (polymer BC of Table VI).

b) A series of additional latices are prepared by following the procedures and using a recipe similar to the one described in part a) of this example except that either a different monomer of Table I is used and/or methyl methacrylate (MMA) is substituted for ethyl acrylate (EA) and/or the monomer ratios are changed. The aqueous viscosities upon neutralization with one equivalent of sodium hydroxide at 1% polymer solids are shown in Table VI.

TABLE VI

| Polymer | Monomer | Wt. ratio | 1% Viscosity[1] mPa · s |
|---|---|---|---|
| BG | #3/EA/MAA | 7.5/52.5/40 | 23,000 |
| BH | #3/EA/MAA | 10/50/40 | 25,000 |
| BJ | #3/EA/MAA | 15/45/40 | 33,500 |
| BK | #4/EA/MAA | 7.5/52.5/40 | 21,000 |
| BL | #4/EA/MAA | 10/50/40 | 20,000 |
| BM | #4/EA/MAA | 15/45/40 | 24,500 |
| BN | #11/EA/MAA | 7.5/52.5/40 | 25,000 |
| BO | #11/EA/MAA | 10/50/40 | 31,000 |
| BP | #11/EA/MAA | 15/45/40 | 30,500 |
| BQ | #10/EA/MAA | 10/50/40 | >50,000[2] |
| BR | #14/MMA/MAA | 37/21.4/41.6 | 7,000 |
| BS | #16/EA/MAA | 10/50/40 | 15,000 |
| BT | #18/EA/MAA | 2/58/40 | 43,000 |
| BU | #17/EA/MAA | 5/55/40 | 23,500 |

[1]Brookfield viscosity at 12 rpm at 23.9°C (75°F), spindle No. 4.
[2]320,000 mPa · s at 1.5 rpm

Example 5

a) An emulsion of monomers in water is prepared by adding 7.1 g of n-dodecyloxy poly(ethyleneoxy)$_{30}$ethyl methacrylate, 134.5 g of ethyl acrylate, 94.4 g of methacrylic acid, 0.236 g of n-dodecyl mercaptan, and 11.8 g of 30% solution of anionic surfactant Alipal EP-110 (ammonium salt of nonylphenoxypoly(ethyleneoxy)$_9$ethanol (sulfate), and 271 g of water. To a reaction vessel containing 11.8 g of 30% solution of Alipal EP-110 in 206 g of water at 85°C there is added 5% of the monomer emulsion and 203 g of 1.25% ammonium persulfate solution. After the initial charge has polymerized at 85°C, the remaining monomer emulsion and 8.7 g of 1.25% ammonium persulfate solution are gradually added over a period of one hour at 85°C. After completion of monomer and initiator feed, the mixture is held at 85°C for 15 minutes and then 30 g of 0.3% ammonium persulfate solution was added. After another 15 minutes hold at 85°C, the mixture is cooled and filtered. The filtrate is an approximately 30% solids emulsion copolymer dispersion in which the copolymer composition is 3% n-dodecyloxy poly(ethyleneoxy)$_{30}$ethyl methacrylate, 57% ethyl acrylate, and 40% methacrylic acid.

b) A series of additional latices was prepared by following procedures similar to the one specifically described in Example 5 a) but varying the hydrophobe-containing monomer type and use level, and chain transfer agent size and level. Table VII lists the compositions.

TABLE VII

| Polymer | Monomer | Weight ratio | Mercaptan[2] | Wt.[3]% |
|---|---|---|---|---|
| BV | #41/EA/MAA | 5/55/40 | TDM | 0.05 |
| BW | #42/EA/MAA | 1/59/40 | DM | 0.086 |
| BX | #43/EA/MAA | 1/59/40 | TDM | 0.68 |
| BY | #42/EA/MAA | 5/55/40 | TDM | 0.114 |
| BZ | #42/EA/MAA | 5/55/40 | DM | 0.52 |
| DD | #43/EA/MAA | 5/55/40 | TDM | 0.05 |
| DE | #44/EA/MAA | 3/47/50 | DDM | 0.1 |
| DF | #44/EA/MAA | 3/67/30 | DDM | 0.1 |
| DF | #45/EA/MAA | 3/57/40 | DDM | 0.1 |
| DH | #45/EA/MAA | 2/58/40 | none | — |
| DI | #46/EA/MAA | 3/57/40 | none | — |
| DJ | #46/EA/MAA | 3/57/40 | DDM | 0.1 |
| DK | #46/EA/MAA | 3/57/40 | DDM | 0.35 |
| DL | #46/EA/MAA | 3/57/40 | DDM | 0.6 |
| DM | #46/EA/MAA | 1/59/40 | DDM | 0.35 |
| DN | #46/EA/MAA | 5/55/40 | DDM | 0.35 |
| DO | #46/EA/MAA | 3/47/50 | DDM | 0.1 |
| DP | #46/EA/MAA | 3/67/30 | DDM | 0.1 |
| DQ | #47/EA/MAA | 2/58/40 | DDM | 0.1 |
| DR | #47/EA/MAA | 2/58/40 | DDM | 0.3 |
| DS | #47/EA/MAA | 2.5/57.5/40 | DDM | 0.2 |
| DT | #47/EA/MAA | 3/57/40 | DDM | 0.1 |
| DU | #47/EA/MAA | 3/57/40 | DDM | 0.3 |
| DV | #48/EA/MAA | 1/59/40 | TDM | 0.05 |
| DW | #48/EA/MAA | 1/59/40 | TDM | 0.114 |
| DX | #48/EA/MAA | 1/59/40 | DM | 0.52 |
| DY | #48/EA/MAA | 3/57/40 | DDM | 0.35 |
| DZ | #48/EA/MAA | 5/55/40 | DM | 0.086 |
| EE | #48/EA/MAA | 5/55/40 | TDM | 0.68 |
| EF | #49/EA/MAA | 3/57/40 | DDM | 0.1 |
| EG | #50/EA/MAA | 3/57/40 | DDM | 0.1 |
| EH | #52/EA/MAA | 3/57/40 | — | |
| EI | #47/EA/MAA | 2/58/40 | — | |

[1]Example 2 a)
[2]TDM=n-tetradecylmercaptan
DDM=n-dodecylmercaptan
DM=n-decylmercaptan
[3]Based on the total weight of the monomers.

Example 6
Pigment printing composition

A water-in-oil clear concentrate is prepared by dissolving 5 parts of a surfactant, sorbitan monooleate, in 30 parts by weight of mineral spirits (a hydrocarbon distillate cut having a flash point over 102°C), then adding while stirring 58 parts of one of the MAA emulsion polymers of the present invention, Polymer C (Table II herein), at 30% solids and mixing therein 7 parts of 28% ammonium hydroxide to at least partially neutralize the polymer dispersion and thereby thicken it. The resulting composition is useful as a clear concentrate that can be diluted with about 95 to 98 parts of water to form a printing clear. Such a clear can then be mixed with an aqueous emulsion polymer to serve as a binder and, if desired, with a color concentrate. The commercial practice to date has been to prepare water-in-oil types of clear concentrates commonly used for the textile printing and dyeing industry by mixing a dusty, powdered thickener, such as certain dry products of U.S. patent 2,798,053 with the other ingredients. The use of polycarboxylic acid thickeners in the form of aqueous emulsion polymer dispersions for preparation of printing compositions for the textile printing and dyeing industry, though suggested in the British patent (page 6, lines 58 to 70), has not been accepted commercially, persumably because of inadequate thickening efficiency obtained by the use of such emulsion polymer dispersions and/or the lack of reliable viscosity expectations in the event of adventitious presence of cations, such as sodium, calcium, magnesium, which may be present in hard water used or in the case of sodium, present in a softened water in an amount that varies in dependence upon variation in the hardness of the water treated or upon unreliable deionization. The possibility of adventitious occurrence of such cations, especially that of sodium is particularly rampant in the commercially available pigment or color concentrates. The acid emulsion copolymers of the present invention provide a more

efficient thickening effect and are less sensitive to the presence, adventitiously or otherwise, of such cations.

The polycarboxylic acid emulsion copolymers of the present invention not only have exceptional thickening efficiency as compared to those of the British patent, but it has been found that they are less sensitive to sodium ions, such as may occur in various small amounts adventitiously in the clear and color concentrates commonly used in the textile printing industry.

Example 7

a) A printing clear is prepared by dissolving sorbitan monooleates (0.06 part) in mineral spirits (1.45 parts), mixing therewith 2.5 parts of polymer BC (Table VI) at 30% solids, 95.49 parts of water and 0.5 part of aqueous 28% ammonium hydroxide.

b) Eighty parts of the clear obtained in part a) is mixed with 10 parts of a binder consisting of a (35% solids) aqueous dispersion of an acrylic emulsion polymer having a heat-reactive component, such as an N-methylol(meth)acrylamide copolymerized therein to enable curing by heat of the composition after application to a textile fabric. This "printing clear" having no colorant therein is designated A.

c) Additional printing pastes are prepared by mixing 90 parts of the clear obtained in part a) with 10 parts of the same 35%-solids binder as in part b) but in one paste 3 parts of the commercially available color concentrate Aqua Hue Blue BGG 9521 is mixed in yielding a paste herein designated C, whereas in another 10 parts of Aqua Hue Blue BGG 9521 is mixed in, yielding a paste herein designated D.

d) The Brookfield Viscosity of the three compositions, A, C, D, are measured at 23.9°C (75°F) using spindle No. 6 at 20 rpm with the following results:

| | |
|---|---|
| A | 19,500 mPa · s |
| C | 32,000 mPa · s |
| D | 46,000 mPa · s |

e) A commercially available clear concentrate is prepared by mixing 15 parts of a powdery polycarboxylic acid copolymer, such as is disclosed in U.S. patent 2,798,053, into a solution of 5 parts sorbitan monooleate in 30 parts of mineral spirits, adding a mixture of about 38.5 parts of water and 7.5 parts of 28% ammonium hydroxide, and finally about 4 parts of a nonionic (oil-in-water) surfactant, such as nonylphenoxypoly(9)ethoxyethanol. Three parts of the clear concentrate is mixed with 97 parts of water to provide a printing clear which is termed herein a 3% commercial clear (or "CC-3%" for simplicity).

f) The "CC-3%" is mixed with the same 35%-solids binder as is used in part b) in the relative proportions of 80 parts of the former with 10 parts of the binder to produce a printing clear.

g) Then additional printing pastes are prepared by adding 3 parts, in one instance, and 10 parts in another instance, of the same blue colour concentrate, Aqua Hue Blue BGG-9521 used in part c) to 90 parts of the printing clear obtained in part f).

Brookfield viscosities of the clear and of the two pastes having corresponding levels of blue as in the clear A of part b) and in pastes C and D of part c) herein are shown in the following table: (A, C, and D are used in this table to indicate level of blue colorant).

TABLE VIII
Printing paste viscosity[1], mPa · s

| | New | Old |
|---|---|---|
| A | 19,500 | 12,000 |
| C | 32,000 | 8,750 |
| D | 46,000 | 7,250 |

[1]Brookfield (model RVF) spindle 6 at 20 rpm and room temperature 23.9°C (75°F)

Table VIII shows that introducing the color increases the viscosity in the printing compositions of the invention, (second column), whereas in the printing compositions made with a commercial clear (third column), the introduction of the color reduces the viscosity.

h) When corresponding printing clears and pastes are made with five other binders (35%-solids acrylic emulsion polymers having reactive components) commonly used in the pigment printing and dyeing of textiles, essentially the same viscosity pattern is shown, increase in viscosity as the amount of blue colorant is increased in the new pastes of the invention and a decrease in viscosity with added colorant in the prior (or "old") pastes.

The clear and colored pastes obtained in parts b), c), f), g) and h) of this example are applied to textile fabrics by the screen-printing process and then cured by heating in conventional ovens. Along

with increased viscosity when the color is added in increasing proportions, the printed textile shows a greater sharpness of the mark, reduction of, or even freedom from flushing and haloing, and better print hold-out with resulting brighter and deeper shade and softer hand.

Example 8

Pigment pastes are prepared using various color concentrates from the printing clear A obtained in accordance with Examples 7 b). Besides preparing pastes with the color levels of C and D as described in example 8 c), an additional paste having a lesser level of color concentrate is made in accordance with the following print paste code or chart:

| Print paste code | A | B | C | D |
|---|---|---|---|---|
| Printing clear | 80 | 80 | 80 | 80 |
| Binder (35% solids) | 10 | 10 | 10 | 10 |
| Color concentrate | — | 0.6 | 3 | 10 |

The following commercially available colorants are used in place of the Aqua Hue Blue BGG 9521, each of the following being used to produce printing pastes having color levels corresponding to B, C, and D:

1. Magenta W-5030
2. Aqua-Hue Scarlet BYDC
3. Helizarine Red BN
4. Blue 2G
5. Blue 3G Type W

The Brookfield RVF viscosity, mPa · s, using spindle 6/20 rpm measured at room temperature on each paste is provided in Table IX following:

TABLE IX

| Pigment | Color level | Viscosity mPa · s |
|---|---|---|
| 1. Magenta | B | 22,000 |
| | C | 25,500 |
| | D | 40,000 |
| 2. Scarlet | B | 26,000 |
| | C | 28,000 |
| | D | 37,500 |
| 3. Helizarine Red | B | 20,000 |
| | C | 16,000 |
| | D | 11,500 |
| 4. Blue 2G | B | 25,000 |
| | C | 27,000 |
| | D | 28,500 |
| 5. Blue 3G Type W | B | 20,750 |
| | C | 19,250 |
| | D | 13,500 |

The data shown in Table IX indicate that colors 3 and 5 do not increase in viscosity as the color is increased, but the others follow the pattern discussed in the preceding example.

Example 9

The printing clear obtained in Example 7 a) is mixed with a commercially available clear made by the procedure described in Example 7 e) wherein the thickener (powdered) is the polycarboxylic acid polymer available under the designation "Carbopol[R] 846" from B. F. Goodrich Corporation. Any proportions may be used, but in this particular example 40 parts of the clear obtained in Example 7 a) is mixed with 40 parts of the clear obtained in Example 7 e) and ten parts of the 35% solids emulsion polymer binder, such as that used in parts c) and f) of Example 7 and a color concentrate in any amount desired, e.g., 0.5 to 4 parts. In Table X, the viscosities of print pastes thereby obtained using Aqua Hue Blue BGG 9521 and having color levels of A, B, C, D in the code or chart hereinabove are noted.

TABLE X

| Color level | Viscosity, mPa · s* |
|---|---|
| A | 41,500 |
| B | 33,000 |
| C | 36,000 |
| D | 35,500 |

*Brookfield RVF, spindle 6/20 rpm

Example 10

Example 9 is repeated using a similar 35% solids acrylic emulsion copolymer dispersion the copolymer having a higher apparent second order transition temperature (Tg). Table XI lists the viscosities.

TABLE XI

| Color level | Viscosity, mPa · s* |
|---|---|
| A | 41,000 |
| B | 25,500 |
| C | 28,000 |
| D | 35,750 |

*Brookfield RVF, spindle 6/20 rpm

Example 11

The pastes produced in Example 9 and 10 are used for printing textile fabrics by the silk-screen process and the printed fabrics are cured in customary fashion by heating. Sharp (mark) prints are obtained and the increase in viscosity as the levels of color increase from B through D result in the benefits associated therewith as mentioned in the discussion presented hereinabove in part h) of Example 7.

Example 12

A dentrifrice is prepared using Polymer C of Table II by mixing 1.7 parts of a 30% solids dispersion of the methacrylic acid copolymer (Polymer C) into a mixture of 20 parts sorbitol and 1.5 parts of sodium lauryl sulfate. Then 0.07 part of sodium hydroxide is mixed in thoroughly to thicken the mixture and 50 parts of finely-divided calcium carbonate is mixed in, water (about 27 parts) being mixed in gradually to form a uniform paste having a Brookfield viscosity (0.8 rpm/23.9°C (75°F) of over 2,000,000 mPa · s.

Example 13

A hand lotion is prepared by mixing 48.5 parts of glycerine with 1.7 parts of a 30% solids dispersion of a methacrylic acid copolymer (Polymer C, Table II or Polymer E, Table II), adding 0.5 part triethanolamine while stirring and adding about 50.5 parts of water gradually to form an unctuous liquid having a viscosity of 35,000 to 40,000 mPa · s.

Example 14

An all-purpose liquid detergent for household use is made by mixing the following ingredients in the proportions (parts) and in the order specified in the table:

| | |
|---|---|
| Tetrapotassium pyrophosphate | 1.0 |
| 2-Butoxyethanol | 2.0 |
| Triton® X-100 (octylphenoxypolyethoxyethanol from Rohm & Haas) | 1.0 |
| Polymer C (30% solids) | 1.3 |
| NaOH | 0.2 |
| Water | 94.5 |

Example 15

A "fracturing" fluid for stimulating the production of oil from oil-wells may simply be made up by mixing one part of a 25% to 30% solids dispersion of one of the polymers of the invention listed in Table II or in Table IV, or in Table VI such as Polymer C, E, AB, AL, BB, BD, or BF with about 0.04 part of NaOH and sufficient water to make a total of 100 parts.

Example 16

a) An emulsion copolymer in water is prepared essentially as described in Example 4, part (a),

except that 23.6 g. of monomer 8 of Table II is used in place of monomer 4. The copolymer composition is 10% stearyloxypoly(ethyleneoxy)$_{20}$ethyl methacrylate, 50% ethyl acrylate, and 40% methacrylic acid.

b) The cothickening effect of added surfactant is shown in Tables XII (anionic) and XIII (nonionic). The data was obtained by blending in water the emulsion copolymer dispersion of a) above and the surfactant and then neutralizing with one equivalent of sodium hydroxide. Viscosities were measured at 23.9°C (75°F) using a Brookfield viscometer. The data illustrates a high level of viscosity enhancement, viscosity maxima as a function of copolymer/surfactant ratios, and a shear thinning effect at higher rpm. Viscosity units are mPa · s (centipoises).

TABLE XII

| % Copolymer | % solids Surfactant[1] | 0.5 rpm | 5 rpm | 0.3 rpm | 3 rpm |
|---|---|---|---|---|---|
| 0.25 | 0 | | | 1,500 | 450 |
| 0.25 | 0.05 | | | 24,000 | 3,180 |
| 0.25 | 0.10 | | | 55,250 | 8,300 |
| 0.25 | 0.15 | | | 40,000 | 11,600 |
| 0.25 | 0.20 | | | 17,250 | 7,750 |
| 0.25 | 0.25 | | | 6,500 | 2,830 |
| 0.50 | 0 | 44,000 | 6,700 | | |
| 0.50 | 0.10 | 295,000 | 46,000 | | |
| 0.50 | 0.15 | 380,000 | 66,000 | | |
| 0.50 | 0.20 | 360,000 | 88,000 | | |
| 0.50 | 0.25 | 250,000 | 66,000 | | |
| 0.50 | 0.30 | 200,000 | 56,000 | | |

[1]Sodium lauryl sulfate

TABLE XIII

| % Copolymer solids | % surfactant[1] | 0.3 rpm | 3 rpm | 30 rpm |
|---|---|---|---|---|
| 0.25 | 0 | 1,500 | 450 | 150 |
| 0.25 | 0.3 | 5,800 | 2,600 | 570 |
| 0.25 | 0.5 | 2,800 | 860 | 320 |

[1]Octylphenoxypolyethoxy ethanol of 12—13 oxyethylene units

Example 17

Variations of the composition of the hydrophobe-containing monomer component of the emulsion copolymer composition of the invention are set forth in Table XIV which follows:

TABLE XIV

| Thickener composition | Use level g/dm$^3$ | Ku Eq. Init/ Unsh/Sh | ICI Init/Equ. |
|---|---|---|---|
| 3 Monomer No. 49 MA/57 EA/40 MAA/0.1 DDM | 8.75 | 82/102/91 | 1.55/1.75 |
| 3 Monomer No. 12 MA/57 EA/40 MAA/0.1 DDM | 9.35 | 81/102/95 | 1.55/1.7 |
| 3 Monomer No. 50 MA/57 EA/40 MAA/0.1 DDM | 8.27 | 83/110/102 | 1.65/1.7 |
| 2 Monomer No. 2/58 MMA/40 MAA/0.2 DDM | 13.3 | 69/72/71 | 1.55/1.45 |
| 2 Monomer No. 22/58 MMA/40 MAA/0.2 DDM | 9.71 | 72/82/82 | 1.5/1.5 |

| Thickener composition | Build* | Flow/ sag | Alkali resistance 60%/% ret. | Gloss 20°/60° |
|---|---|---|---|---|
| 3 Monomer No. 49 MA/57 EA/40 MAA/0.1 DDM | 12.0 | 7+/9 | 4/95 | 51/84 |
| 3 Monomer No. 12 MA/57 EA/40 MAA/0.1 DDM | 12.5 | 7+/8+ | 5/94 | 48/85 |
| 3 Monomer No. 50 MA/57 EA/40 MAA/0.1 DDM | 14.5 | 8/7+ | 1/99 | 58/88 |
| 2 Monomer No. 2/58 MMA/40 MAA/0.2 DDM | 9.5 | 8/7 | 1/97 | 44/81 |
| 2 Monomer No. 22/58 MMA/40 MAA/0.2 DDM | 8.0 | 7+/9 | | 41/82 |

Init = Initial Unsh = Unsheared Sh = Sheared Equ. = Equilibrated

*Build is thickness of coating applied by conventional means in gram per sq.ft. (0.093 m$^2$) of coated surface.

**Claims for the contracting states: BE, CH, DE, FR, GB, IT, NL, SE**

1. A thickener or thickened composition containing (A) water-insoluble copolymer thickener made by emulsion polymerization and containing
(1) 20 to 69.5 weight percent of at least one of methacrylic acid and acrylic acid;
(2) 0.5 to 25 weight percent of at least one monomer of the formula

$$H_2C{=}C(R){-}C(O){-}O{-}(CH_2CH_2O)_n{-}R^o \qquad (I)$$

wherein R is H or $CH_3$, n is at least 2 and $R^o$ is a radical having from 8 to 30 carbon atoms and is at least one of substituted or unsubstituted alkyl, alkylaryl, and polycyclic alkyl;
(3) at least 30 weight percent of at least one alkyl acrylate and/or alkyl methacrylate in which the alkyl group has from 1 to 4 carbon atoms, and
(4) zero to 1.0 weight percent of a polyethylenically unsaturated monomer,
the total of the percentages of monomers (1), (2), (3) and, when present, (4) being 100; and (B) surfactant in an amount of 0.05 to 1.0 parts by weight per part of copolymer thickener A solids.

2. A composition as claimed in claim 1 wherein copolymer A is a copolymer of:
(1) 30 to 50 weight percent of methacrylic acid,
(2) 2 to 25 weight percent of at least one monomer of the formula

$$H_2C{=}C(R){-}C(O){-}O{-}(CH_2CH_2O)_n{-}R^o \qquad (I)$$

wherein R is H or $CH_3$, n is at least 2 and has an average value up to 60 or more, and $R^o$ is an alkyl group having from 12 to 24 carbon atoms,
(3) at least 30 weight percent of alkyl acrylate in which the alkyl group has from 1 to 4 carbon atoms, and
(4) zero to 1.0 weight percent of a polyethylenically unsaturated monomer,
the total of the percentages of components (1), (2), (3) and, when present, (4) amounting to 100.

3. A composition as claimed in claim 1 in which the proportion of component (1) is from 30 to 45 weight percent, the proportion of component (2) is from 1 to 15 weight percent, and the proportion of component (3) is from 40 to 60 weight percent.

4. A composition as claimed in any of claims 1 to 3 where, in the formula of component (2), R is $CH_3$ and $R^o$ is an alkyl group having from 12 to 20 carbons atoms.

5. A composition as claimed in any of claims 1 to 4 where, in the formula of component (2), n is at least 10.

6. A composition as claimed in any of claims 1, 3, 4 or 5 wherein the copolymer A has the composition:
(1) 30 to 45 weight percent of methacrylic acid,
(2) 1 to 15 weight percent of at least one monomer of the formula

$$H_2C{=}C(CH_3){-}C(O){-}O{-}(CH_2CH_2O)_n{-}R^o$$

wherein n has an average value of from 10 to 60 and $R^o$ is an alkyl group having from 12 to 18 carbon atoms, and
(3) 40 to 50 weight percent of ethyl acrylate.

7. A composition as claimed in any preceding claim in which the copolymer A is at least partially neutralized.

8. A composition as claimed in any preceding claim wherein the surfactant is anionic or nonionic.

9. An aqueous dispersion of a composition according to any preceding claim.

10. An aqueous dispersion as claimed in claim 9 which also contains pigment.

11. An aqueous dispersion as claimed in claim 10 in the form of a water based paint containing pigment in a pigment volume concentration of up to 65% and vinyl addition emulsion polymer binder comprising at least one of vinyl acetate polymer, polymer of acrylic and/or methacrylic acid ester, and styrenebutadiene polymer.

12. A method of thickening an aqueous system which comprises providing in the system a composition as claimed in claim 7.

13. A method as claimed in claim 12 which comprises adding copolymer and surfactant according to any of claims 1 to 6 separately or jointly to an aqueous system and at least partially neutralizing the copolymer.

14. Use of a thickened or thickener composition according to any of claims 1 to 7 in a latex paint, pigment dispersion, oil well drilling fluid, textile printing paste, dentifrice, hand lotion, pharmaceutical or liquid detergent.

15. Use of a copolymer A as defined in any of Claims 1 to 6 in a dentifrice, pigment printing composition, household liquid detergent formulation, hand lotion, pharmaceutical or fracturing fluid.

**Claims for the contracting state: AT**

1. A process for preparing a thickened or thickener composition which comprises mixing (A) an emulsion polymerized thickener copolymer containing

(1) 20 to 69.5 weight percent of at least one of methacrylic acid and acrylic acid;

(2) 0.5 to 25 weight percent of at least one monomer of the formula

$$H_2C{=}C(R){-}C(O){-}O{-}(CH_2CH_2O)_n{-}R^o \qquad (I)$$

wherein R is H or $CH_3$, n is at least 2 and $R^o$ is a radical having from 8 to 30 carbon atoms and is at least one of substituted or unsubstituted alkyl, alkylaryl, and polycyclic alkyl;

(3) at least 30 weight percent of at least one alkyl acrylate and/or alkyl methacrylate in which the alkyl group has from 1 to 4 carbon atoms, and

(4) zero to 1.0 weight percent of a polyethylenically unsaturated monomer,

the total of the percentages of monomers (1), (2), (3) and, when present (4) being 100, with (B) 0.05 to 1.0 parts by weight per part by weight of thickener copolymer (A) solids of surfactant.

2. A process as claimed in Claim 1 wherein the copolymer is a copolymer of:

(1) 30 to 50 weight percent of methacrylic acid,

(2) 2 to 25 weight percent of at least one monomer of the formula

$$H_2C{=}C(R){-}C(O){-}O{-}(CH_2CH_2O)_n{-}R^o \qquad (I)$$

wherein R is H or $CH_3$, n is at least 2 and has an average value up to 60 or more, and $R^o$ is an alkyl group having from 12 to 24 carbon atoms.

(3) at least 30 weight percent of an alkyl acrylate in which the alkyl group has from 1 to 4 carbon atoms, and

(4) zero to 1.0 weight percent of a polyethylenically unsaturated monomer,

the total of the percentages of components (1), (2), (3) and, when present, (4) amounting to 100.

3. A process as claimed in Claim 1 in which the proportion of component (1) is from 30 to 45 weight percent, the proportion of component (2) is from 1 to 15 weight percent, and the proportion of component (3) is from 40 to 60 weight percent.

4. A process as claimed in any of Claims 1 to 3 where, in the formula of component (2), R is $CH_3$ and $R^o$ is an alkyl group having from 12 to 20 carbon atoms.

5. A process as claimed in any of Claims 1 to 4 where, in the formula of component (2), n is at least 10.

6. A process as claimed in any of Claims 1, 3, 4 or 5 wherein the copolymer (A) has the composition:

(1) 30 to 45 weight percent of methacrylic acid,

(2) 1 to 15 weight percent of at least one monomer of the formula

$$H_2C{=}C(CH_3){-}C(O){-}O{-}(CH_2CH_2O)_n{-}R^o$$

wherein n has an average value of from 10 to 60 and $R^o$ is an alkyl group having from 12 to 18 carbon atoms, and

(3) 40 to 60 weight percent of ethyl acrylate.

7. A process as claimed in any preceding claim wherein the copolymer is at least partially neutralized.

8. A process as claimed in any preceding claim wherein the surfactant is anionic or nonionic.

9. A process as claimed in any preceding claim wherein the copolymer and surfactant are mixed into the form of an aqueous dispersion.

10. A process as claimed in Claim 9 wherein pigment is also mixed into the dispersion.

11. A process as claimed in Claim 10 for making a water based paint containing pigment in a pigment volume concentration of up to 65% and vinyl addition emulsion polymer binder comprising at least one of vinyl acetate polymer, polymer of acrylic and/or methacrylic acid ester, and styrenebutadiene polymer.

12. A method of thickening an aqueous system which comprises providing in the system at least partially neutralized thickener copolymer as defined in any of Claims 1 to 6 and, at least one surfactant in an amount of 0.05 to 1.0 parts by weight per part by weight of thickener copolymer (A) solids.

13. A method as claimed in Claim 12 which comprises adding surfactant and thickener copolymer to an aqueous system and at least partially neutralizing the copolymer.

14. Use of a thickened or thickener composition prepared according to any preceding claim in a latex paint, pigment dispersion, oil well drilling fluid, textile printing paste, dentrifice, hand lotion, pharmaceutical or liquid detergent.

15. Use of a copolymer A as defined in any of Claims 1 to 6 in a dentifrice, pigment printing composition, household liquid detergent formulation, hand lotion, pharmaceutical or fracturing fluid.

**Patentansprüche für die Vertragsstaaten: BE, CH, DE, FR, GB, IT, NL, SE**

1. Eindickungsmasse oder eingedickte Masse, die (A) ein wasserunlösliches Copolymereindickungsmittel, hergestellt durch Emulsionspolymerisation und enthaltend

1. 20 bis 69,5 Gew.-% wenigstens eines der Komponenten Methacrylsäure und Acrylsäure,
2. 0,5 bis 25 Gew.-% wenigstens eines Monomeren der Formel

$$H_2C{=}C(R){-}C(O){-}O{-}(CH_2CH_2O)_n{-}R^o$$

worin R für H oder $CH_3$ steht, n wenigstens 2 bedeutet und $R^o$ ein Rest mit 8 bis 30 Kohlenstoffatomen ist und wenigstens aus einem substituierten oder nichtsubstituierten Alkylrest, Alkylarylrest oder polycyclischen Alkylrest besteht,

3. wenigstens 30 Gew.-% wenigstens eines Alkylacrylats und/oder Alkylmethacrylats, in welchem die Alkylgruppe 1 bis 4 Kohlenstoffatome aufweist und
4. 0 bis 1,0 Gew.-% eines polyethylenisch ungesättigten Monomeren,

wobei die gesamten Prozentsätze der Monomeren (1), (2), (3) und, falls vorhanden (4) 100 betragen, und (B) ein grenzflächenaktives Mittel in einer Menge von 0,05 bis 1,0 Gew.-Teilen pro Teil der Feststoffe des Copolymereindickungsmittels A enthält.

2. Masse nach Anspruch 1, dadurch gekennzeichnet, daß das Copolymere A ein Copolymeres aus

1. 30 bis 50 Gew.-% Methacrylsäure,
2. 2 bis 25 Gew.-% wenigstens einem Monomeren der Formel

$$H_2C{=}C(R){-}C(O){-}O{-}(CH_2CH_2O)_n{-}R^o$$

worin R für H oder $CH_3$ steht, n wenigstens 2 bedeutet und einen Durchschnittswert von bis zu 60 oder mehr besitzt, und $R^o$ eine Alkylgruppe mit 12 bis 24 Kohlenstoffatomen darstellt,

3. wenigstens 30 Gew.-% Alkylacrylat, wobei die Alkylgruppe 1 bis 4 Kohlenstoffatome aufweist, und
4. 0 bis 1,0 Gew.-% eines polyethylenisch ungesättigten Monomeren ist, wobei die Prozentmengen der Komponenten (1), (2) und (3) und, falls vorhanden (4) sich auf 100 belaufen.

3. Masse nach Anspruch 1, dadurch gekennzeichnet, daß die Menge der Komponente (1) 30 bis 45 Gew.-%, die Menge der Komponente (2) 1 bis 15 Gew.-% und die Menge der Komponente (3) 40 bis 60 Gew.-% beträgt.

4. Masse nach einem der Ansprüche 1 bis 3, dadurch gekennzeichnet, daß in der Formel der Komponente (2) R für $CH_3$ steht und $R^o$ eine Alkylgruppe mit 12 bis 20 Kohlenstoffatomen ist.

5. Masse nach einem der Ansprüche 1 bis 4, dadurch gekennzeichnet, daß in der Formel für die Komponente (2) n wenigstens 10 beträgt.

6. Masse nach einem der Ansprüche 1, 3, 4 oder 5, dadurch gekennzeichnet, daß das Copolymere A sich wie folgt zusammensetzt:

1. aus 30 bis 45 Gew.-% Methacrylsäure,
2. aus 1 bis 15 Gew.-% wenigstens einem Monomeren der Formel

$$H_2C{=}C(CH_3){-}C(O){-}O{-}(CH_2CH_2O)_n{-}R^o$$

worin n einen durchschnittlichen Wert von 10 bis 60 besitzt und $R^o$ eine Alkylgruppe mit 12 bis 18 Kohlenstoffatomen bedeutet, und

3. 40 bis 50 Gew.-% Ethylacrylat.

7. Masse nach einem der vorhergehenden Ansprüche, dadurch gekennzeichnet, daß das Copolymere A wenigstens teilweise neutralisiert ist.

8. Masse nach einem der vorhergehenden Ansprüche, dadurch gekennzeichnet, daß das grenzflächenaktive Mittel anionisch oder nichtionisch ist.

9. Wäßrige Dispersion einer Masse gemäß einem der vorhergehenden Ansprüche.

10. Wäßrige Dispersion gemäß Anspruch 9, dadurch gekennzeichnet, daß sie auch ein Pigment enthält.

11. Wäßrige Dispersion nach Anspruch 10 in Form eines Anstrichmittels auf Wasserbasis, die ein Pigment in einer Pigmentvolumenkonzentration von bis zu 65% und ein Vinyladditionsemulsionspolymerbindemittel aus wenigstens einem der Bestandteile Vinylacetatpolymerem, einem Polymeren aus einem Acrylsäure- und/oder Methacrylsäureester und einem Styrol/Butadien-Polymeren enthält.

12. Verfahren zur Eindickung eines wäßrigen Systems, dadurch gekennzeichnet, daß in das System eine Masse gemäß Anspruch 7 eingebracht wird.

13. Verfahren nach Anspruch 12, dadurch gekennzeichnet, daß das Copolymere und das grenzflächenaktive Mittel gemäß einem der Ansprüche 1 bis 6 getrennt oder gleichzeitig einem wäßrigen System zugesetzt werden und das Copolymer wenigstens teilweise neutralisiert wird.

14. Verwendung einer eingedickten Masse oder einer Eindickungsmasse nach einem der Ansprüche 1 bis 7 in einem Latexanstrichmittel, einer Pigmentdispersion, einem Ölquellenbohrfluid,

einer Textildruckpaste, einer Zahnpasta, einer Handlotion, einem pharmazeutischen oder flüssigen Detergens.

15. Verwendung eines Copolymeren A gemäß einem der Ansprüche 1 bis 6 in einer Zahnpasta, einer Pigmentdrückmasse, einer flüssigen Haushaltsdetergensformulierung, einer Handlotion, eines Pharmazeutikums oder einem Frakturierungsfluid.

**Patentansprüche für den Vertragsstaat: AT**

1. Verfahren zur Herstellung einer eingedickten Masse oder Eindickungsmasse, dadurch gekennzeichnet, daß vermischt werden (A) ein emulsionspolymerisiertes Eindickungscopolymeres, enthaltend

(1) 20 bis 69,5 Gew.-% wenigstens einen der Bestandteile Methacrylsäure und Acrylsäure,

(2) 0,5 bis 25 Gew.-% wenigstens ein Monomeres der Formel

$$H_2C{=}C(R){-}C(O){-}O{-}(CH_2CH_2O)_n{-}R^o \qquad (I)$$

worin R für H oder $CH_3$ steht, n wenigstens 2 bedeutet und $R^o$ einen Rest mit 8 bis 30 Kohlenstoffatomen ist, wobei es sich um wenigstens eine substituierte oder nichtsubstituierte Alkyl-, Alkylaryl- oder polycyclische Alkylgruppe handelt.

(3) wenigstens 30 Gew.-% wenigstens eines Alkylacrylats und/oder Alkylmethacrylats, in welchem die Alkylgruppe 1 bis 4 Kohlenstoffatome aufweist und

(4) 0 bis 1,0 Gew.-% eines polyethylenisch ungesättigten Monomeren,

wobei die Gesamtmenge der Prozentsätze der Monomeren (1), (2), (3) und, falls vorhanden, (4) 100 beträgt mit (B) 0,05 bis 1,0 Gew.-Teilen pro Gew.-Teil der Eindickungscopolymerfeststoffe (A) eines grenzflächenaktiven Mittels.

2. Verfahren nach Anspruch 1, dadurch gekennzeichnet, daß das Copolymere ein Copolymeres ist aus

(1) 30 bis 50 Gew.-% Methacrylsäure,

(2) 2 bis 25 Gew.-% wenigstens eines Monomeren der Formel

$$H_2C{=}C(R){-}C(O){-}O{-}(CH_2CH_2O)_n{-}R^o \qquad (I)$$

worin R für H oder $CH_3$ steht, n wenigstens 2 bedeutet und einen durchschnittlichen Wert von bis zu 60 oder mehr besitzt und $R^o$ eine Alkylgruppe mit 12 bis 24 Kohlenstoffatomen darstellt,

(3) wenigstens 30 Gew.-% eines Alkylacrylats, in welchem die Alkylgruppe 1 bis 4 Kohlenstoffatome besitzt, und

(4) 0 bis 1,0 Gew.-% eines polyethylenisch ungesättigten Monomeren,

wobei die Gesamtmenge der Prozentsätze der Komponenten (1), (2) und (3) und, falls vorhanden, (4) 100 beträgt.

3. Verfahren nach Anspruch 1, dadurch gekennzeichnet, daß die Menge der Komponente (1) 30 bis 45 Gew.-%, die Menge der Komponente (2) 1 bis 15 Gew.-% und die Menge der Komponente (3) 40 bis 60 Gew.-% beträgt.

4. Verfahren nach einem der Ansprüche 1 bis 3, dadurch gekennzeichnet, daß in der Formel der Komponente (2) R für $CH_3$ steht und $R^o$ eine Alkylgruppe mit 12 bis 20 Kohlenstoffatomen bedeutet.

5. Verfahren nach einem der Ansprüche 1 bis 4, dadurch gekennzeichnet, daß in der Formel der Komponente (2) n wenigstens 10 ist.

6. Verfahren nach einem der Ansprüche 1, 3, 4 oder 5, dadurch gekennzeichnet, daß das Copolymere (A) folgende Zusammensetzung besitzt:

(1) 30 bis 45 Gew.-% Methacrylsäure,

(2) 1 bis 15 Gew.-% wenigstens eines Monomeren der Formel

$$H_2C{=}C(CH_3){-}C(O){-}O{-}(CH_2CH_2O)_n{-}R^o,$$

worin n einen durchschnittlichen Wert von 10 bis 60 besitzt und $R^o$ eine Alkylgruppe mit 12 bis 18 Kohlenstoffatomen ist, und

(3) 40 bis 60 Gew.-% Ethylacrylat.

7. Verfahren nach einem der vorhergehenden Ansprüche, dadurch gekennzeichnet, daß das Copolymere wenigstens teilweise neutralisiert ist.

8. Verfahren nach einem der vorhergehenden Ansprüche, dadurch gekennzeichnet, daß das grenzflächenaktive Mittel anionisch oder nichtionisch ist.

9. Verfahren nach einem der vorhergehenden Ansprüche, dadurch gekennzeichnet, daß das Copolymere und das grenzflächenaktive Mittel in Form einer wäßrigen Dispersion vermischt werden.

10. Verfahren nach Anspruch 9, dadurch gekennzeichnet, daß ein Pigment in die Dispersion eingemischt wird.

11. Verfahren nach Anspruch 10 zur Herstellung eines Anstrichmittels auf Wasserbasis, das ein Pigment in einer Pigmentvolumenkonzentration von bis zu 65% und ein Vinyladditionsemulsionspolymerbindemittel aus wenigstens einem Vinylacetatpolymeren, einem Polymeren eines Acrylsäure- und/oder Methacrylsäureesters und einem Styrol/Butadien-Polymeren enthält.

12. Verfahren zum Eindicken eines wäßrigen Systems, dadurch gekennzeichnet, daß in das System ein wenigstens teilweise neutralisiertes Eindickungscopolymeres gemäß einem der Ansprüche 1 bis 6 und wenigstens ein grenzflächenaktives Mittel in einer Menge von 0,05 bis 1,0 Gew.-Teilen pro Gewichtsteil der Eindickungscopolymerfeststoffe (A) eingebracht wird.

13. Verfahren nach Anspruch 12, dadurch gekennzeichnet, daß das grenzflächenaktive Mittel und das Eindickungscopolymere zu einem wäßrigen System zugesetzt werden und das Copolymere wenigstens teilweise neutralisiert wird.

14. Verwendung einer eingedickten Masse oder einer Eindikkungsmasse, hergestellt nach einem der vorhergehenden Ansprüche, in einem Latexanstrichmittel, einer Pigmentdispersion, einem Ölquellenborfluid, einer Textildruckpaste, einer Zahnpasta, einer Handlotion, einem Pharmazeutikum oder einem flüssigen Detergens.

15. Verwendung eines Copolymeren A gemäß einem der Ansprüche 1 bis 6 in einer Zahnpasta, einer Pigmentdruckmasse, einer flüssigen Haushaltsdetergensformulierung, einer Handlotion, eines Pharmazeutikums oder einem Frakturierungsfluid.

**Revendications pour les Etats Contractants: BE, CH, DE, FR, GB, IT, NL, SE**

1. Une composition d'épaississant ou épaissie contenant (A) un copolymère épaississant insoluble dans l'eau fait par polymérisation en émulsion et contentant

(1) 20 à 69,5% en poids d'au moins un acide parmi l'acide méthacrylique et l'acide acrylique;

(2) 0,5 à 25% en poids d'au moins un monomère de formule

$$H_2C{=}C(R){-}C(O){-}O{-}(CH_2CH_2O)_n{-}R^o \qquad (I)$$

dans laquelle R est H ou $CH_3$, n est au moins 2 et $R^o$ est un radical ayant 8 à 30 atomes de carbone et est au moins un alcoyle, alcoylaryle et alcoyle polycyclique substitués ou non substitués;

(3) au moins 30% en poids d'au moins un acrylate d'alcoyle et/ou méthacrylate d'alcoyle dont le radical alcoyle a de 1 à 4 atomes de carbone; et

(4) 0 à 1,0% en poids d'un monomère à insaturation polyéthylénique,

le total des pourcentages de monomères (1), (2), (3) et, lorsqu'il est présent, (4) étant 100; et (B) un agent tensio-actif en une quantité de 0,05 à 1,0 partie en poids par partie des matières sèches du copolymère épaississant A.

2. Une composition comme revendiquée dans la revendication 1, dans laquelle le copolymère A est un copolymère de:

(1) 30 à 50% en poids d'acide méthacrylique,

(2) 2 à 25% en poids d'au moins un monomère de formule

$$H_2C{=}C(R){-}C(O){-}O{-}(CH_2CH_2O)_n{-}R^o \qquad (I)$$

dans laquelle R est H ou $CH_3$, n est au moins 2 et a une valeur moyenne allant jusqu'à 60 ou plus et $R^o$ est un radical alcoyle ayant 12 à 24 atomes de carbone,

(3) au moins 30% en poids d'acrylate d'alcoyle dont le radical alcoyle a de 1 à 4 atomes de carbone, et

(4) 0 à 1,0% en poids d'un monomère à insaturation polyéthylénique,

le total des pourcentages des composés (1), (2), (3) et, lorsqu'il est présent, (4) atteignant 100.

3. Une composition comme revendiquée dans la revendication 1, dans laquelle la proportion du composant (1) est de 30 à 45% en poids, la proportion du composant (2) est de 1 à 15% en poids et la proportion du composant (3) est de 40 à 60% en poids.

4. Une composition comme revendiquée dans l'une quelconque des revendications 1 à 3, dans laquelle dans la formule du composant (2) R est $CH_3$ et $R^o$ est un radical alcoyle ayant 12 à 20 atomes de carbone.

5. Une composition comme revendiquée dans l'une quelconque des revendications 1 à 4, dans laquelle dans la formule du composant (2) n est au moins 10.

6. Une composition comme revendiquée dans l'une quelconque des revendications 1, 3, 4 ou 5 dans laquelle le copolymère A a pour composition:

(1) 30 à 45% en poids d'acide méthacrylique,

(2) 1 à 15% en poids d'au moins un monomère de formule

$$H_2C{=}C(CH_3){-}C(O){-}O{-}(CH_2CH_2O)_n{-}R^o$$

dans laquelle n a une valeur moyenne de 10 à 60 et $R^o$ est un radical alcoyle ayant 12 à 18 atomes de carbone, et

(3) 40 à 50% en poids d'acrylate d'éthyle.

7. Une composition comme revendiquée dans une revendication précédente quelconque, dans laquelle le copolymère A est au moins partiellement neutralisé.

8. Une composition comme revendiquée dans une revendication précédente quelconque, dans laquelle l'agent tensio-actif est anionique ou non ionique.

9. Une dispersion aqueuse d'une composition selon une revendication précédente quelconque.

10. Une dispersion aqueuse comme revendiquée dans la revendication 9 qui contient également un pigment.

11. Une dispersion aqueuse comme revendiquée dans la revendication 10, sous forme d'une peinture à base aqueuse contenant un pigment avec un concentration pigmentaire volumique allant jusqu'à 65% et un copolymère d'émulsion d'addition vinylique, liant, comprenant au moins un polymère d'acétate de vinyle, un polymère d'ester d'acide acrylique et/ou méthacrylique et un polymère de styrène-butadiène.

12. Un procédé d'épaississement d'un système aqueux qui comprend l'apport au système d'une composition comme revendiquée dans la revendication 7.

13. Un procédé comme revendiqué dans la revendication 12, qui comprend l'addition d'un copolymère et d'un agent tensio-actif selon l'une quelconque des revendications 1 à 6, séparément ou conjointement à un système aqueux et la neutralisation au moins partielle du copolymère.

14. Emploi d'une composition épaissie ou d'épaississant selon l'une quelconque des revendications 1 à 7, dans une peinture au latex, une dispersion de pigment, un fluide de forage pour puits de pétrole, une pâte d'impression des textiles, un dentifrice, une lotion pour les mains, un produit pharmaceutique ou un détergent liquide.

15. Emploi d'un copolymère A comme défini dans l'une quelconque des revendications 1 à 6, dans un dentifrice, une composition d'impression pigmentaire, une composition de détergent liquide domestique, une lotion pour les mains, un produit pharmaceutique ou un liquide de fracturation.

**Revendications pour l'Etat contractant: AT**

1. Un procédé pour la préparation d'une composition d'épaississant ou épaissie qui comprend le mélange de (A) un copolymère épaississant polymérisé en émulsion contentant

(1) 20 à 69,5% en poids d'au moins un acide parmi l'acide méthacrylique et l'acide acrylique;

(2) 0,5 à 25% en poids d'au moins un monomère de formule

$$H_2C{=}C(R){-}C(O){-}O{-}(CH_2CH_2O)_n{-}R^o \qquad (I)$$

dans laquelle R est H ou $CH_3$, n est au moins 2 et $R^o$ est un radical ayant de 8 à 30 atomes de carbone et est au moins un alcoyle, alcoylaryle et alcoyle polycyclique substitués ou non substitués;

(3) au moins 30% en poids d'au moins un acrylate d'alcoyle et/ou méthacrylate d'alcoyle dont le radical alcoyle a de 1 à 4 atomes de carbone, et

(4) 0 à 1,0% en poids d'un monomère à insaturation polyéthylénique,

le total des pourcentages des monomères (1), (2), (3) et, lorsqu'il est présent, (4) étant de 100, avec (B) 0,05 à 1,0 partie en poids d'agent tensio-actif par partie en poids de matières sèches du copolymère épaississant (A).

2. Un procédé comme revendiqué dans la revendication 1, dans lequel le copolymère est un copolymère de

(1) 30 à 50% en poids d'acide méthacrylique,

(2) 2 à 25% en poids d'au moins un monomère de formule

$$H_2C{=}C(R){-}C(O){-}O{-}(CH_2CH_2O)_n{-}R^o \qquad (I)$$

dans laquelle R est H ou $CH_3$, n est au moins 2 et a une valeur moyenne allant jusqu'à 60 ou plus, et $R^o$ est un radical alcoyle ayant 12 à 24 atomes de carbone,

(3) au moins 30% en poids d'un acrylate d'alcoyle dont le radical alcoyle a de 1 à 4 atomes de carbone et

(4) 0 à 1,0% en poids d'un monomère à insaturation polyéthylenique,

le total des pourcentages des composants (1), (2), (3) et, lorsqu'il est présent, (4) atteignant 100.

3. Un procédé comme revendiqué dans la revendication 1, dans lequel la proportion du composant (1) est de 30 à 45% en poids, la proportion du composant (2) est de 1 à 15% en poids et la proportion du composant (3) est de 40 à 60% en poids.

4. Un procédé comme revendiqué dans l'une quelconque des revendications 1 à 3, dans lequel, dans la formule du composant (2), R est $CH_3$ et $R^o$ est un radical alcoyle ayant 12 à 20 atomes de carbone.

5. Un procédé comme revendiqué dans l'une quelconque des revendications 1 à 4, dans lequel, dans la formule du composant (2), n est au moins 10.

6. Un procédé comme revendiqué dans l'une quelconque des revendications 1, 3, 4 ou 5, dans lequel le copolymère (A) a pour composition:

(1) 30 à 45% en poids d'acide méthacrylique,

(2) 1 à 15% en poids d'au moins un monomère de formule

$$H_2C{=}C(CH_3){-}C(O){-}O{-}(CH_2CH_2O)_n{-}R^o$$

dans laquelle n a une valeur moyenne de 10 à 60 et $R^o$ est un radical alcoyle ayant de 12 à 18 atomes de carboné, et

(3) 40 à 60% en poids d'acrylate d'éthyle.

7. Un procédé comme revendiqué dans une revendication précédente quelconque, dans lequel le copolymère est au moins partiellement neutralisé.

8. Un procédé comme revendiqué dans une revendication précédente quelconque, dans lequel l'agent tensio-actif est anionique ou non ionique.

9. Un procédé comme revendiqué dans une revendication précédente quelconque, dans lequel le copolymère et l'agent tensio-actif sont mélangés sous forme d'une dispersion aqueuse.

10. Un procédé comme revendiqué dans la revendication 9, dans lequel un pigment est également mélangé à la dispersion.

11. Un procédé comme revendiqué dans la revendication 10, pour préparer une peinture à base aqueuse contenant un pigment avec une concentration pigmentaire volumique allant jusqu'à 65% et un polymère d'émulsion d'addition vinylique, liant, comprenant au moins un polymère d'acétate de vinyle, un polymère d'ester d'acide acrylique et/ou d'acide méthacrylique et un polymère de styrène-butadiène.

12. Un procédé d'épaississement d'un système aqueux qui comprend l'apport dans le système d'un copolymère épaississant au moins partiellement neutralisé comme défini dans l'une quelconque des revendications 1 à et d'au moins un agent tensio-actif en une quantité de 0,05 à 1,0 partie en poids par partie en poids des matières sèches du copolymère épaississant (A).

13. Un procédé comme revendiqué dans la revendication 12, qui comprend l'addition d'un agent tensio-actif et d'un copolymère épaississant à un système aqueux et la neutralisation au moins partielle du copolymère.

14. Emploi d'une composition épaissie ou d'épaississant préparée selon une revendication précédente quelconque, dans une peinture au latex, une dispersion de pigment, un fluide de forage de puits de pétrole, une pâte d'impression des textiles, un dentifrice, une lotion pour les mains, un produit pharmaceutique ou un détergent liquide.

15. Emploi d'un copolymère A comme défini dans l'une quelconque des revendications 1 à 6, dans un dentifrice, une composition d'impression pigmentaire, une composition de détergent liquide domestique, une lotion pour les mains, un produit pharmaceutique ou un liquide de fracturation.